# EUROPÄISCHE PATENTANMELDUNG

(11) **EP 0 945 427 A1**
(43) Veröffentlichungstag der Anmeldung: **29.09.1999**
(21) Anmeldenummer: 99104959.4
(22) Anmeldetag: 12.03.1999
(51) Int. Cl.: C07C 211/36, C07C 265/10, C07C 215/44, C07C 49/747, C07C 39/17

(54) **Cycloaliphatische Verbindungen als Polymerbausteine**

(30) Priorität: 23.03.1998 DE 19812613
(71) Anmelder: Bayer Aktiengesellschaft, 51368 Leverkusen (DE)
(72) Erfinder: Jautelat, Manfred, Dr., 51399 Burscheid (DE); Casser, Carl, Dr., 13585 Berlin (DE); Müller, Hanns-Peter, Dr., 51519 Odenthal (DE); Hajek, Manfred, Dr., 51375 Leverkusen (DE)

(57) **Zusammenfassung**

Gegenstand der Erfindung sind neue cycloaliphatische Verbindungen ausgewählt aus der Formel 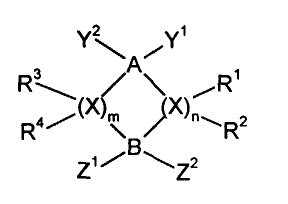 worin
R¹, R², R³ und R⁴ für jedes X individuell wählbar, unabhängig voneinander Wasserstoff, C₁-C₆-Alkyl, C₅-C₆-Cycloalkyl, Phenyl und Halogen,
m und n unabhängig voneinander für die Zahl 2, 3, 4 oder 5 stehen, und
X, A und B Kohlenstoff und
Y¹, Y², Z¹ und Z² individuell wählbar, unabhängig voneinander Wasserstoff, Hydroxy (OH), Amino (NH₂), Isocyanato (NCO) oder 4-Hydroxyphenyl bedeuten.

## Beschreibung

Gegenstand der Erfindung sind neue cycloaliphatische Verbindungen der Formel (I), 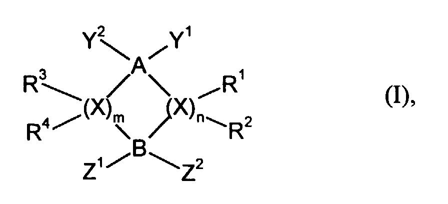 worin
R¹, R², R³ und R⁴ für jedes X individuell wählbar, unabhängig voneinander Wasserstoff, C₁-C₆-Alkyl, C₅-C₆-Cycloalkyl, Phenyl und Halogen,
m und n unabhängig voneinander für die Zahl von 2, 3, 4 oder 5 stehen und
X, A und B Kohlenstoff und
Y¹, Y², Z¹ und Z² individuell wählbar, unabhängig voneinander Wasserstoff, Hydroxy (OH), Amino (NH₂), Isocyanato (NCO) oder 4-Hydroxyphenyl bedeuten
mit der Maßgabe, daß an mindestens einem Atom X eines der Paare R¹ und R² oder R³ und R⁴ gleichzeitig Alkyl ist und
mit der Maßgabe, daß wenn Y¹ und Y² jeweils gleich 4-Hydroxyphenyl bedeuten, Z¹ Hydroxy (OH) und Z² Wasserstoff oder Z¹ und Z² gemeinsam eine Oxogruppe (=O) bedeuten, und wenn einer der Reste Y¹, Y², Z¹ oder Z² für Isocyanato (NCO) steht, die übrigen Gruppen nicht Hydroxyl (OH), Amimo (NH₂) oder 4-Hydroxyphenyl bedeuten und wenn Y¹ oder Y² Hydroxy (OH) bedeuten, Z¹ und Z² nicht Wasserstoff (H) bedeuten und wenn Z¹ und Z² Hydroxy (OH) bedeuten, Y¹ und Y² nicht Hydroxy (OH) oder Wasserstoff (H) bedeuten und für den Fall, daß Z¹, Z², Y¹ und Y² nicht für 4-Hydroxyphenyl stehen, zwei Gruppen Z¹, Z², Y¹ und Y² für Hydroxyl(OH) oder Amino (NH₂) stehen.

Bevorzugt sind an 1 bis 2 Atomen X, insbesondere nur an einem Atom X, R¹ und R² bzw. R³ und R⁴ gleichzeitig Alkyl. Bevorzugter Alkylrest ist Methyl. Bevorzugt ist, daß die Summe aus n und m vier ist und insbesondere m und n jeweils zwei ist. Besonders bevorzugt ist, daß m und n jeweils zwei ist (für die Zahl 2 stehen) und dabei unter der obengenannten Maßgabe nur die X-Atome in α-Stellung zu einem Kohlenstoffatom A oder B alkylsubstituiert sind. Bevorzugt ist weiterhin, daß ein Kohlenstoffatom A oder B mit 4-Hydroxyphenyl disubstituiert ist. Vorzugsweise sind die Verbindungen bifunktionell, z.B. enthalten sie zwei NCO-reaktive Gruppen oder zwei NCO-Gruppen.

Bevorzugte Cycloalkanderivate entsprechen beispielsweise den Formeln 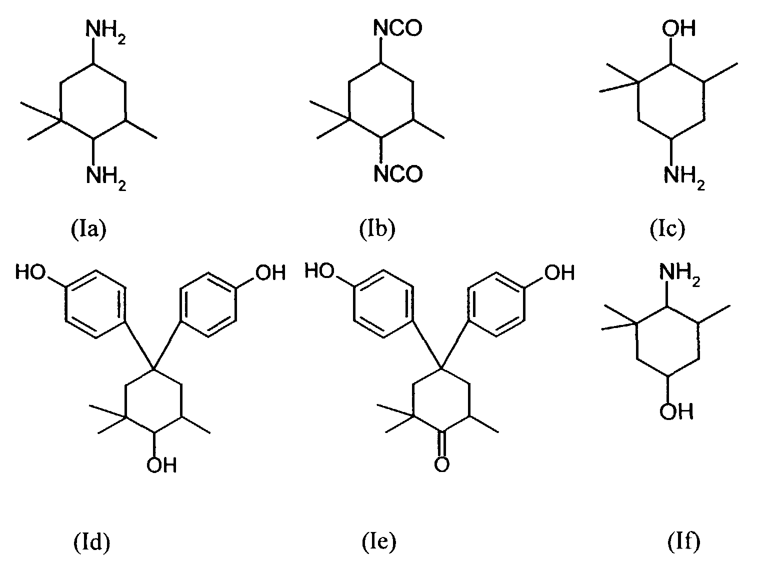 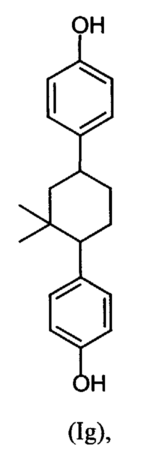 wobei das 1,4-Diamino-2,2,6-trimethylcyclohexan (Formel Ia), das 1,4-Diisocyanato-2,2,6-trimethylcyclohexan (Formel Ib), das 1-Hydroxy-2,2,6-trimethyl-4-aminocyclohexan (Formel Ic), das 1,1-Bis-(4-hydroxyphenyl)-3,3,5-trimethyl-4-hydroxycyclohexan (Formel Id) und das 1,1-Bis-(4-hydroxyphenyl)-3,3,5-trimethyl-4-oxocyclohexan (Formel Ie) besonders bevorzugt sind.

Die erfindungsgemäßen Verbindungen der Formel (I) können in an sich bekannter Weise hergestellt werden.

So erhält man z.B. durch reduktive Aminierung der Diketone der Formel (II) oder (III) 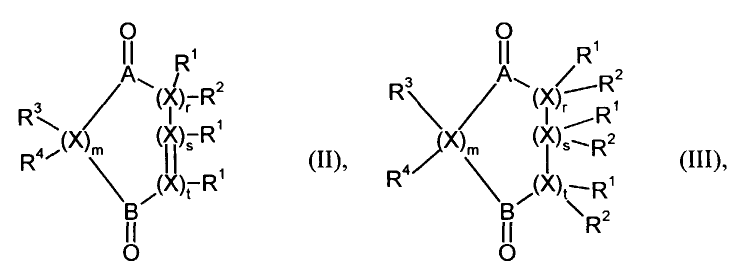 worin
R¹, R², R³, R⁴, X, A, B, m und die für Formel (I) angegebene Bedeutung haben und
r, s und t jeweils unabhängig voneinander die Zahl 0, 1, 2, 3, 4 oder 5 bedeuten,
mit der Maßgabe, daß die Summe von r, s und t die Zahl n ergibt,
die Diaminoverbindungen der allgemeinen Formel (I), worin R¹, R², R³ R⁴, A, B, X, m und n die für Formel (I) angegebene Bedeutung haben und Y¹ und Z¹ gleichzeitig Amino (NH₂) und Y² und Z² gleichzeitig Wasserstoff bedeuten und
gegebenenfalls auch die Aminoalkohole der allgemeinen Formel (IV) 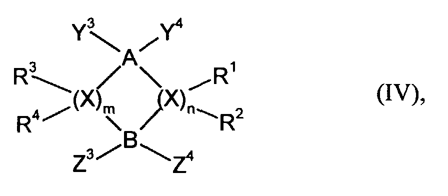 worin
R¹, R², R³, R⁴, m, n, X, A und B die für Formel (I) angegebene Bedeutung haben und
Y³ und Z³ Wasserstoff bedeuten und
Y⁴ und Z⁴ jeweils unabhängig voneinander Hydroxy (OH) oder Amino (NH₂) bedeuten.

Die Aminoalkohole der Formel (IV) werden ebenfalls durch reduktive Aminierung der Hydroxyketone der allgemeinen Formel (V) oder (VI) 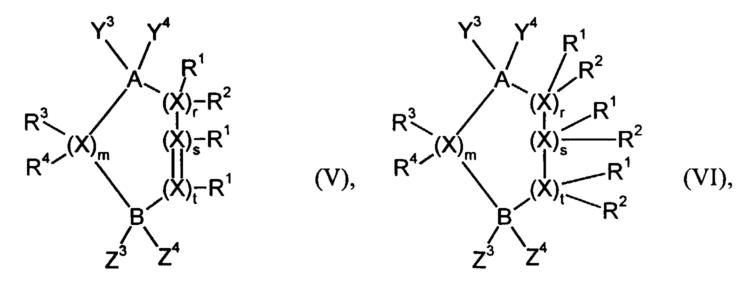 worin
R¹, R², R³, R⁴, X, A, B und m die für Formel (I) angegebene Bedeutung haben und
r, s und t jeweils unabhängig voneinander die Zahl von 0, 1, 2, 3, 4 oder 5 bedeuten,
mit der Maßgabe, daß die Summe aus r, s und t die Zahl n ergibt, und
Y³ und Y⁴ zusammen eine Oxogruppe (=O) und dabei gleichzeitig Z³ Hydroxy (OH) und Z⁴ Wasserstoff oder
Z³ und Z⁴ zusammen eine Oxogruppe (=O) und dabei gleichzeitig Y³ Hydroxy und Y⁴ Wasserstoff bedeuten.

Die Diketone der Formeln (II), (III) sowie die Hydroxyketone der Formel (V) und (VI) sind literaturbekannt (siehe beispielsweise) K. Mori, Tetrahedron Vol. 30, 1065-1072 (1974), J.N. Marx, F. Sondheimer, Tetrajedron, Supp. 8, Part I, 1-7 (1966), H.G.W. Leuenberger, W. Boguth, E. Widmer, R. Zell, Helveticfa Chimica Acta, Vol. 59, 1832-1849, (1976) und DE-A 24 57 158.

Für Einzelheiten der reduktiven Aminierung von Ketonen sei auf W. Schneider, K. Lehmann in Methodicum Chimicum, Band 6, Seite 536 ff. und auf R. Schröter, F. Möller in Houben-Weyl, Band XI/1, Seite 602 ff. verwiesen. Weitere Einzelheiten zur Durchführung der reduktiven Aminierung finden sich in den Patentschriften DE 30 31 955, 30 11 656, 12 26 078 und 40 33 609.

Besonders bevorzugte Diketone der Formel (II) und (III) und besonders bevorzugte Hydroxyketone der Formel (V) und (VI) sind die Verbindungen der Formeln

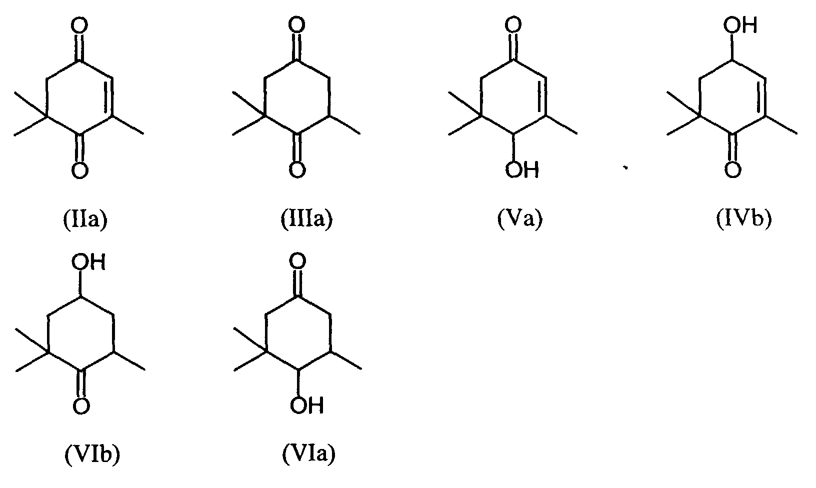

Die Aminoalkohole der allgemeinen Formel (IV) lassen sich auch durch katalytische Hydrierung der Imine der allgemeinen Formel (VII) oder (VIII) 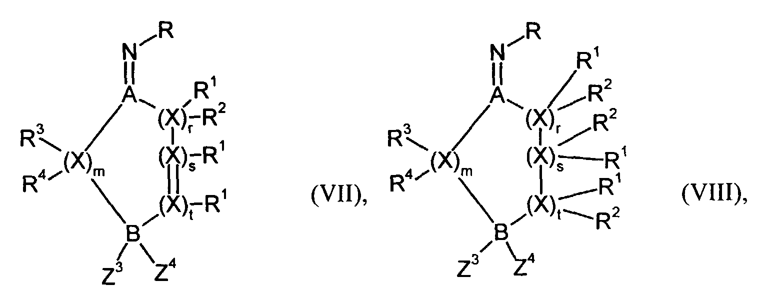 worin
R¹, R², R³, R⁴, X, A, B und m die für Formel (I), (II), (III), (V) und (VI) angegebene Bedeutung haben und
r, s und t jeweils unabhängig voneinander die Zahl 0, 1, 2, 3, 4 oder 5 bedeuten,
mit der Maßgabe, daß die Summe aus r, s und t die Zahl n ergibt und
Z³ Wasserstoff und gleichzeitig Z⁴ Hydroxy (OH) bedeutet oder
Z³ und Z⁴ gemeinsam eine Oxogruppe (=O) bedeuten und
R für Wasserstoff, Benzyl, Hydroxy oder für den Rest der allgemeinen Formel (IX) oder (X) 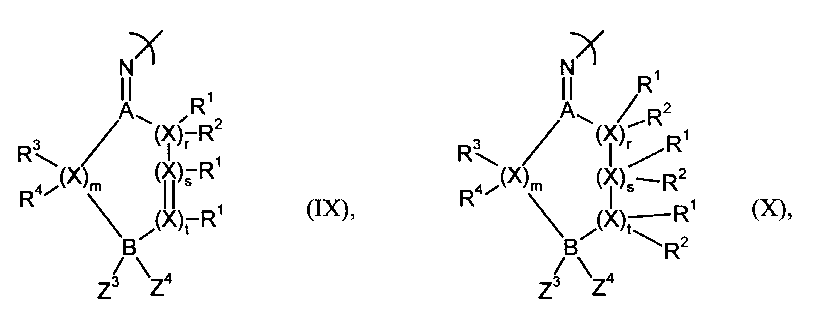 worin
R¹, R², R³, R⁴, X, A, B, m, Z¹, Z², r, s und t die für Formel (VII) und (VIII) angegebene Bedeutung haben,
   steht,
   erhalten.

Ebenso sind durch reduktive Aminierung der Imine der allgemeinen Formel (VII) oder (VIII), worin R¹, R², R³, R⁴, A, B, X, m, r, s, t und R die für Formel (XI) angegebene Bedeutung haben und Z³ und Z⁴ gemeinsam eine Oxogruppe (=O) bedeuten, die Diamine der allgemeinen Formel (I), worin R¹, R², R³, R⁴, A, B, X, m und n die für Formel (I) angegebene Bedeutung haben und Y¹ und Z¹ gleichzeitig Amino (NH₂) und gleichzeitig Y² und Z² Wasserstoff bedeuten erhältlich.

Die Imine der allgemeinen Formel (VII) und (VIII) sind nach literaturbekannten Methoden durch Umsetzung der Ketone der allgemeinen Formel (II), (III), (V) und (VI) mit Aminderivaten wie z.B. Ammoniak, Hydrazin, Hydroxylamin, Benzylamin zugänglich (siehe beispielsweise Houben-Weyl, Band X/2, Seite 85 ff. und Band 7/2b, Seite 1944 ff.).

Besonders bevorzugte Imine der allgemeinen Formel (VII) und (VIII) sind die Verbindungen

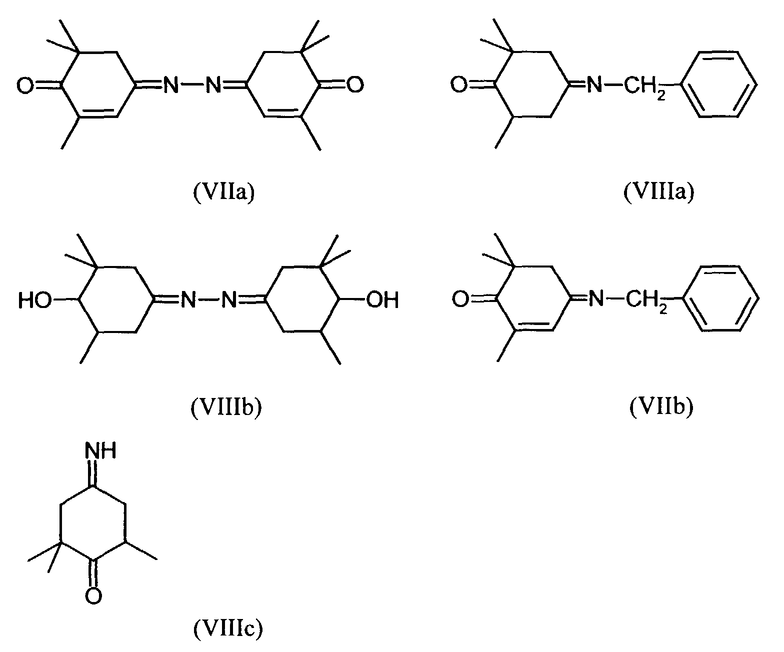

Ausgehend von den Diaminoverbindungen der allgemeinen Formel (I), worin R¹, R², R³, R⁴, A, B, X, m und n die für Formel (I) angegebene Bedeutung haben und Y¹ und Z¹ gleichzeitig Amino (NH₂) und gleichzeitig Y² und Z² Wasserstoff bedeuten, lassen sich durch Umsetzung mit Phosgen nach literaturbekannten Methoden (siehe beispielsweise Houben-Weyl, Band VIII, Seite 119 ff.) die Diisocyanatoverbindungen der allgemeinen Formel (I), worin R¹, R², R³, R⁴, A, B, X, m und n die für Formel (I) angegebene Bedeutung haben und Y¹ und Z¹ gleichzeitig Isocyanato (NCO) und gleichzeitig Y² und Z² Wasserstoff bedeuten, darstellen.

Die Dihydroxydiphenylcycloalkane der allgemeinen Formel (I) 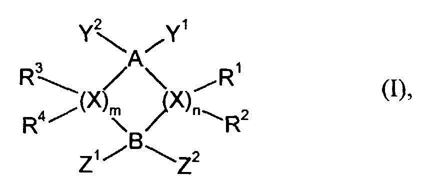 worin
R¹, R², R³, R⁴, X, A, B, m und n die für Formel (I) und (II) angegebene Bedeutung haben und
Y¹ und Y² gleichzeitig 4-Hydroxyphenyl bedeuten und
Z¹ Wasserstoff und gleichzeitig Z² Hydroxy (OH) bedeutet oder
Z¹ und Z² gemeinsam eine Oxogruppierung (=O) bedeuten,
können in an sich bekannter Weise durch Kondensation von Phenol mit Ketonen der allgemeinen Formel (II) und (V), worin R¹, R², R³, R⁴, A, B, X, Y¹, Y², Z¹, Z², m und r die für die Formeln (II) und (V) angegebene Bedreutung haben und s und t die ganze Zahl Null bedeuten, in Gegenwart saurer Katalysatoren und gegebenenfalls durch Zugabe von schwefelhaltigen Co-Katalysatoren hergestellt werden. Für Einzelheiten dieser Kondensation sei auf Schnell, Chemistry and Physics of Polycarbonates, Interscience Publishers, New York 1964 sowie z.B. auf die EP-A 359 953 und die US-A 52 10 328 verwiesen.

Die erfindungsgemäßen Verbindungen der allgemeinen Formel (I) sind wertvolle Zwischenprodukte zur Herstellung und Modifizierung von Polymeren wie Polycarbonaten, Polyamiden, Polyharnstoffen und Polyurethanen usw. (siehe beispielsweise Houben-Weyl, Band E20, Teil 2).

### Beispiele

### Beispiel 1

### 1,4-Diamino-2,2,6-trimethylcyclohexan (Formel (Ia)

In einem 3 l Autoklaven werden bei Raumtemperatur eine Mischung aus 152 g 4-Oxoisophoron (Formel IIa), 150 g Ammoniumsulfat, 50 g Ra/Ni und 1 l Ammotuak mit 80 bar Wasserstoff beaufschlagt und anschließend 20h bei 180°C gerührt. Nach beendeter Wasserstoffaufnahme wird das Reaktionsgemisch entspannt, in Ethanol aufgenommen, filtriert und fraktioniert destilliert. Man erhält 130 g Diamin der Formel (Ia) mit einem Siedepunkt von 62 bis 65°C bei 0,1 mbar.

### Beispiel 2

### 1,4-Diamino-2,2,6-trimethylcyclohexan (Formel Ia)

Zu 152 g 4-Oxoisophoron (Formel IIa) in 125 ml Ethanol gibt man innerhalb von 30 min 25 g Hydrazinhydrat in 30 ml Ethanol. Man laßt das Reaktionsgemisch 12 h unter Rückfluß kochen, engt auf das halbe Volumen ein und filtriert das ausgefallene Ketazin-Produkt (133 g, Fp. 132°C) der Formel (VIIa). Dieses Ketazinderivat (VIIa) kann ohne weitere Reinigung für weitere Umsetzungen verwendet werden.

150 g Ketazinderivat (VIIa), 140 g Ammoniumsulfat und 25 g Ra/Ni/Co in 1 l Ammoniak werden analog Beispiel 1 hydriert. Hach Aufarbeitung analog Beispiel 1 werden 135 g Diamin der Formel (Ia) erhalten.

### Beispiel 3

### 1-Amino-3,3,5-trimethyl-4-hydroxycyclohexan (Formel Ic)

156 g 3,3,5-Trimethyl-4-hydroxycyclohexanon (Formel VIa), 12 g Ammoniumacetat, 60 g Ra/Ni in 250 ml Methanol werden bei 100°C und 100 bar Wasserstoffdruck in Gegenwart von 250 ml Ammoniak hydriert. Nach beendeter Wasserstoffaufnahme wird vom Katalysator filtriert, im Vakuum eingeengt und der verbleibende Rückstand im Hochvakuum fraktioniert destilliert. Bei Kp.: 90 bis 97°C (0,1 mbar) erhält man 130 g Aminoalkohol der Formel (Ic) mit einem Schmelzpunkt von 110 bis 112°C. Nach gaschromatographischer Untersuchung liegt ein Isomerengemisch (vier Isomere) vor.

### Beispiel 4

### 1-Amino-3,3,5-trimethyl-4-hydroxycyclohexan (Formel Ic)

Eine Lösung aus 60 g Ketazinderivat (Formel VIIa) aus Beispiel 2, 8 ml Triethylamin und 400 ml Methanol wird in Gegenwart von 24 g Ra/Ni bei 140°C und 150 bar Wasserstoffdruck bis zur beendeten Wasserstoffaufnahme hydriert. Nach Aufarbeitung analog Beispiel 3 werden 51 g Aminoalkohol der Formel (Ic) erhalten.

### Beispiel 5

### 1,4-Diisocyanato-3,3,5-trimethylcyclohexan (Formel Ib)

Zu einer Lösung aus 500 g Phosgen in 3 l Chlorbenzol tropft man bei 0 bis 10°C eine Lösung aus 156 g Diamin der Formel (Ia) in 250 g Chlorberzol. Das Reaktionsgemisch wird unter weiterer Phosgeneinleitung auf 120°C erhitzt und bei dieser Temperatur bis zum Klarpunkt phosgeniert. Überschüssiges Phosgen und Chlorbenzol werden im Vakumm abdestilliert und der verbleibende Rückstand fraktioniert destilliert. Bei 84 bis 87°C (0,1 bis 0,3 mbar) werden 167 g Diisocyanat der Formel (Ib) erhalten. Hach ¹H-spektroskopischer Untersuchung wird ein Isomerengemisch aus vier Isomeren erhalten.

### Beispiel 6

### 1,1-Bis-(4-hydroxyphenyl)-3,3,5-trimethyl-4-oxocyclohexan (Formel Ie)

8 g 3,3,5-Trimethyl-1,4-dioxocyclohexan (Formel IIIa), 30 g Phenol und 0,5 ml β-Mercaptopropionsäure werden mit 6 ml konzentrierter Salzsäure versetzt und bei 30°C bis zum vollständigen Umsatz (gaschromatographische Verfolgung) des Diketons gerührt. Überschüssiges Phenol wird mit Wasser extrahiert und der verbleibende Rückstand mehrmals mit Toluol und n-Hexan bei 60°C gewaschen. Es verbleiben 6 g Bisphenol (Fp.: 140°C) der Formel (Ie), IR (KBr): 3600 bis 3100 cm⁻¹ (OH), 1680 cm⁻¹ (CO).

### Beispiel 7

### 1,1 Bis-(4-hydroxyphenyl)-3,3,5-trimethyl-4-hydroxycyclohexan (Formel Id)

Analog zu Beispiel 6 werden anstelle von 8 g Diketon der Formel (IIIa) 10 g Hydroxyketon der Formel (VIa) eingesetzt. Man erhält 5 g Bisphenol der Formel (Id), Fp.:102°C (Zers.). Nach ¹H-spektroskopischer Untersuchung handelt es sich um zwei Isomere (77/23), wobei beim Hauptisomeren die OH-Funktion axial und die 5-CH₃-Gruppe äquatorial angeordnet ist.

### Beispiel 8

Gemäß Beispiel 1 der noch nicht veröffentlichten eigenen Anmeldung DE 19 653 585.9 wurde eine colöserfreie, wäßrige Polyurethandispersion hergestellt.

### Vergleichsbeispiel (DE 19 653 585.9)

In einem Reaktionsgefäß werden 170 g (0,1 mol) eines Polyesters aus Adipinsäure, 1,6-Hexandiol und Neopentylglykol mit einem durchschnittlichen Molekulargewicht von 1 700 g/mol und 2 % OH 30 Minuten bei 120°C und 10 mbar unter Rühren entwässert. Unter Stickstoff trägt man 13,4 g (0,1 Mol) Dimethylolpropionsäure und 111 g (0,5 mol) Isophorondiisocyanat ein. Nach 1 Stunde Reaktionszeit bei 110°C wird der Ansatz auf 60°C abgekühlt und in 100 g Aceton gelöst. Nach Zugabe von 18 g (0,2 mol) 1,4-Butandiol wird 22 Stunden bei 50°C nachgerührt und mit 500 g Aceton verdünnt. Zum NCO-Prepolymer wird bei 50°C eine Mischung aus 10,6 g (0,062 mol) Isophorondiamin, 1,07 g (0,016 mol) 25 %ige Ammoniaklösung und 60 g Wasser gegeben. Anschließend rührt man 5 Stunden bei 50°C nach. Es wird mit 3,4 g (0,05 mol) 25 %iger Ammoniaklösung neutralisiert und mit 450 g Wasser dispergiert. Das Aceton entfernt man bis 50°C und 150 mbar und erhält so eine weiße Dispersion mit einem Feststoffgehalt von 39,2 % und einer mittleren Teilchengröße von 263 nm.

Mit einer 200 µm Rakel wurde auf einer Glasplatte ein Naßfilm gezogen, der anschließend im Trockenscbrank 10 Minuten bei 80°C getrocknet wurde. Hieraus resultierte ein Tockenfilm von 70 bis 80 µm. Der Trockenfilm wurde vom Glas abgezogen und im Zugversuch in Anlehnung an DIN 53 455 untersucht.

### Erfindungsgemaßes Beispiel:

Es wurde genauso vorgegangen wie im Vergleichsbeispiel beschrieben, nur mit dem Unterschied, daß anstelle von 0,062 mol Isophorondiamin 0,062 mol 1,4-Diamino-2,2,6-trimethylcyclohexan (Ia) eingesetzt wurden.

Nach der Herstellung der wäßrigen Dispersion wurde ebenfalls mit einer 200 µm Rakel auf einer Glasplatte ein Naßfilm gezogen, der anschließend im Trockenschrank bei 80°C getrocknet wurde. Hieraus resultierte ebenfalls ein Trockenfilm von 70 bis 80 µm. Der Trockenfilm wurde vom Glas abgezogen und ebenfalls im Zugversuch untersucht.

Bei dem mit dem erfindungsgemäßen Diamin(Ia) hergestellten Film liegen der E-Modul um 30 % und die Streckspannung bzw. Dehnung um 10 % höher als im Vergleich.

### Beispiel 9

67,2 g (0,4 mol) 1,6-Hexamethylendiisocyanat werden in 40 g Aceton gelöst. In diese Lösung werden unter Rühren 15,7 g (0,1 mol) der erfindungsgemäßen Verbindung (Ic) eingetragen. Dabei erwärmt sich der Ansatz bis auf 40°C. Man heizt den Ansatz innerhalb von 75 Minuten auf 66°C auf. Dabei entsteht eine klare Lösung. Anschließend wird Vakuum angelegt und das Aceton abgezogen. Dem Ansatz wird dann eine Probe zur NCO-Bestimung entnommen.

Der NCO-Gehalt der Mischung beträgt 30,3 % NCO. Anschließend wird der Ansatz weitere 30 Minuten unter Stickstoff bei 140 bis 150°C gerührt. Das resultierende modifizierte Polyisocyanat besitzt einen NCO-Gehalt von 22,1 % NCO. Das IR-Spektrum weist auf Biuretbildung hin.

## Patentansprüche

1. Verbindungen der Formel (I) 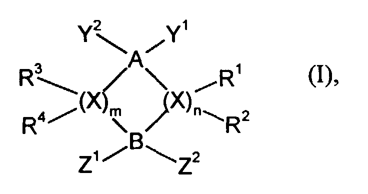 worin
R¹, R², R³ und R⁴ unabhängig voneinander und für jedes X-Atom individuell wählbar Wasserstoff, C₁-C₆-Alkyl, C₅-C₆-Cycloalkyl, Phenyl und Halogen,
m und n unabhängig voneinander die Zahl 2, 3, 4 oder 5 stehen und
X, A und B Kohlenstoff
und
Y¹, Y², Z¹ und Z² individuell wählbar, unabhängig voneinander Wasserstoff, Hydroxy, Amino, Isocyanato oder 4-Hydroxyphenyl
bedeuten,
mit der Maßgabe, daß an mindestens einem Atom X, R¹ und R² oder R³ und R⁴ gleichzeitig Alkyl ist
und
mit der Maßgabe, daß wenn Y¹ und Y² jeweils gleich 4-Hydroxyphenyl bedeuten, Z¹ Hydroxy und Z² gleichzeitig Wasserstoff oder Z¹ und Z² gemeinsam eine Oxogruppe bedeuten wenn einer der Reste Y¹, Y², Z¹ oder Z² für Isocyanato (NCO) steht, die übrigen Gruppen nicht Hydroxy (OH), Amino (NH₂) oder 4-Hydroxyphenyl bedeuten und wenn Y¹ oder Y² Hydroxy (OH) bedeuten, Z¹ und Z² nicht Wasserstoff (H) bedeuten und wenn Z¹ oder Z² Hydroxy (OH) bedeuten, Y¹ und Y² nicht Hydroxy (OH) oder Wasserstoff (H) bedeuten und wenn Z¹, Z², Y¹ und Y⁴ nicht für 4-Hydroxyphenyl stehen, zwei Gruppen Z¹, Z², Y¹ und Y² für Hydroxyl (OH) oder (NH₂) stehen.

2. Verbindungen der Formel (I) gemäß Anspruch 1, dadurch gekennzeichnet, daß in Formel (I) m und n jeweils zwei und R¹, R², R³ und R⁴ für jedes X-Atom individuell wählbar, unabhängig voneinander Wasserstoff und Methyl bedeuten.

3. Verfahren zur Herstellung difunktioneller Verbindungen der Formel (I) gemäß Anspruch 1, worin Y¹, Y², Z¹ und Z² individuell wählbar Wasserstoff, Hydroxy und Amino bedeuten, dadurch gekennzeichnet, daß man Verbindungen der Formeln (V) und (VI) 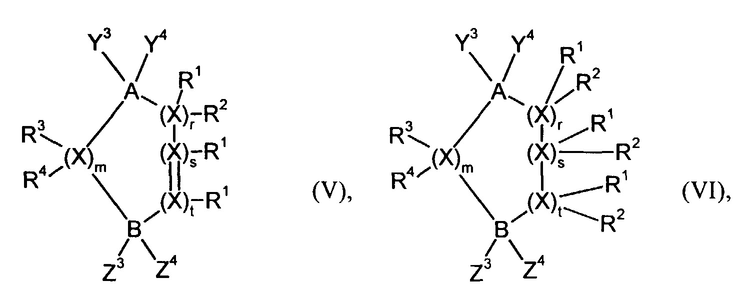 worin
R¹, R², R³, R⁴, X, A, B und m die für Formel (I) angegebene Bedeutung haben und
r, s und t unabhängig voneinander, individuell wählbar die Zahl 0, 1, 2, 3, 4 oder 5 bedeuten, mit der Maßgabe, daß die Summe aus r, s und t die Zahl n ergibt, und
Y³ und Y⁴ sowie Z³ und Z⁴ jeweils gemeinsam eine Oxogruppe bedeuten oder
Y³ und Y⁴ gemeinsam eine Oxogruppe und gleichzeitig Z³ Hydroxy und Z⁴ Wasserstoff bedeuten oder
Z³ und Z⁴ zusammen eine Oxogruppe (=O) und dabei gleichzeitig Y³ Hydroxy und Y⁴ Wasserstoff bedeuten, oder
Y³ und Y⁴ eine Imino (=N-R)-gruppe und gleichzeitig Z³ Hydroxy und Z⁴ Wasserstoff oder Z³ und Z⁴ gemeinsam eine Oxogruppe bedeuten, wobei
R Wasserstoff, Benzyl, Hydroxy oder einen Rest der Formel (IX) oder (X) 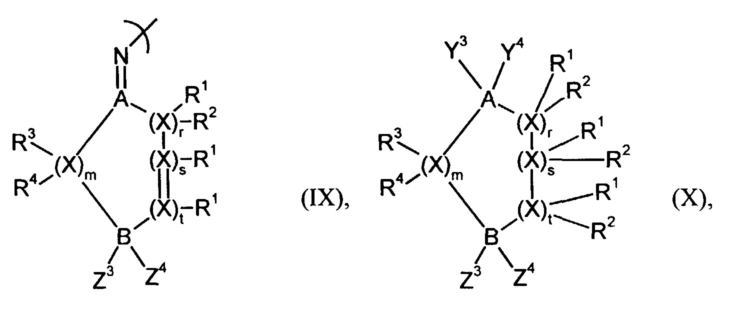 worin
R¹, R², R³, R⁴, X, A, B, m, Z³, Z⁴, Y³, Y⁴r, s und t die für Formel (V) und (VI) angegebene Bedeutung haben,
bedeutet,
mit Wasserstoff und gegebenenfalls mit Ammoniak in Gegenwart eines Hydrierkatalysators, wie z.B. Raney-Nickel, Raney-Kobalt, Pt oder Pd, bei Temperaturen von 50 bis 200°C und bei Drücken von 10 bis 250 bar und gegebenenfalls in Anwesenheit eines Lösungsmittels und/oder wasserentziehender Mittel umsetzt.

4. Verbindung gemäß Anspruch 1, dadurch gekennzeichnet, daß die Verbindung der Formel (I) 1,4-Diamino-2,2,6-trimethylcyclohexan ist.

5. Verbindung gemäß Anspruch 1, dadurch gekennzeichnet, daß die Verbindung der Formel (I) 1,4-Diisocyanato-2,2,6-trimethylcyclohexan ist.

6. Verbindung gemäß Anspruch 1, dadurch gekennzeichnet, daß die Verbindung der Formel (I) 1-Hydroxy-2,2,6-trimethyl-4-aminocyclohexan ist.

7. Verbindung gemäß Anspruch 1, dadurch gekennzeichnet, daß die Verbindung der Formel (I) 1,1-Bis-(4-hydroxyphenyl)-3,3,5-trimethyl-4-oxocyclohexan ist.

8. Verbindung gemaß Anspruch 1, dadurch gekennzeichnet, daß die Verbindung der Formel (I) 1,1-Bis-(4-hydroxyphenyl)-3,3,5-trimethyl-4-hydroxycyclohexan ist.
